(19) Europäisches Patentamt / European Patent Office / Office européen des brevets

(11) **EP 2 637 500 B1**

(12) **FASCICULE DE BREVET EUROPEEN**

(45) Date de publication et mention
de la délivrance du brevet:
**28.02.2018 Bulletin 2018/09**

(21) Numéro de dépôt: **11797364.4**

(22) Date de dépôt: **04.11.2011**

(51) Int Cl.:
*A01N 25/04* (2006.01)          *A01N 63/02* (2006.01)
*A01N 65/00* (2009.01)          *A01N 65/24* (2009.01)
*A01P 1/00* (2006.01)           *A01P 15/00* (2006.01)
*C09D 5/14* (2006.01)           *C09D 11/00* (2014.01)
*A01N 37/02* (2006.01)

(86) Numéro de dépôt international:
**PCT/IB2011/054927**

(87) Numéro de publication internationale:
**WO 2012/063176 (18.05.2012 Gazette 2012/20)**

(54) **COMPOSITIONS FLUIDES APTES À FORMER UN REVÊTEMENT PRÉSENTANT DES PROPRIÉTÉS ANTIVIRALES**

FLÜSSIGE ZUSAMMENSETZUNGEN ZUR HERSTELLUNG EINER BESCHICHTUNG MIT ANTIVIRALEN EIGENSCHAFTEN

LIQUID COMPOSITIONS FOR FORMING A COATING WITH ANTIVIRAL PROPERTIES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **08.11.2010 FR 1059195**

(43) Date de publication de la demande:
**18.09.2013 Bulletin 2013/38**

(73) Titulaire: **Oberthur Fiduciaire SAS**
**75008 Paris (FR)**

(72) Inventeur: **ROSSET, Henri**
**38730 Le Pin (FR)**

(74) Mandataire: **Nony**
**11 rue Saint-Georges**
**75009 Paris (FR)**

(56) Documents cités:
EP-A2- 1 138 314          WO-A1-2005/056449
WO-A1-2006/008566        WO-A2-03/084326
WO-A2-2008/127416        WO-A2-2010/128487
US-A- 4 662 403          US-A- 5 968 538

• **DATABASE WPI Week 201032 Thomson Scientific, London, GB; AN 2010-F02129 XP002660092, & CN 101 698 769 A (FOSHAN FUTIAN COATING CHEM CO LTD) 28 avril 2010 (2010-04-28)**
• **LOIZZO M R ET AL: "Phytochemical Analysis and in vitro Antiviral Activities of the Essential Oils of Seven Lebanon Species", CHEMISTRY & BIODIVERSITY, HELVETICA CHIMICA ACTA, ZUERICH, CH, vol. 5, no. 3, 1 mars 2008 (2008-03-01), pages 461-470, XP002558017, ISSN: 1612-1872, DOI: 10.1002/CBDV.200890045 [extrait le 2008-03-20]**
• **CLARKE N M ET AL: "Effect of antimicrobial factors in human milk on rhinoviruses and milk-bourne cytomegalovirus in vitro", JOURNAL OF MEDICAL MICROBIOLOGY, HARLOW, GB, vol. 49, 30 juin 2000 (2000-06-30), pages 719-723, XP002558016, ISSN: 0022-2615**
• **LUCILLA SEGANTI ET AL: "Antiviral activity of lactoferrin towards naked viruses", BIOMETALS, KLUWER ACADEMIC PUBLISHERS, BO, vol. 17, no. 3, 1 juin 2004 (2004-06-01), pages 295-299, XP019232211, ISSN: 1572-8773, DOI: 10.1023/B:BIOM.0000027708.27142.BC**
• **HALLDOR THORMAR ET AL: "Inactivation of Enveloped Viruses and Killing of Cells by Fatty Acids and Monoglycerides", ANTIMICROBIAL AGENTS AND CHEMOTHERAPY, 1 janvier 1987 (1987-01-01), pages 27-31, XP55018609,**

- Tatsawan Tipvarakarnkoon ET AL: "Rheological properties and phase change behaviors of coconut fats and oils", ANNUAL TRANSACTIONS OF THE NORDIC RHEOLOGY SOCIETY, 1 January 2008 (2008-01-01), pages 1-7, XP55115490, Retrieved from the Internet: URL:http://rheology-esr.net/Open_transacti ons/2008/Posters/Tipvarakarnkoon et al.pdf [retrieved on 2014-04-28]

**Description**

L'invention concerne des compositions fluides aptes à former, en surface de support, un revêtement, en particulier un vernis, une encre, une laque ou une peinture, et avantageusement dotées de propriétés antivirales.

Le domaine de l'invention est plus particulièrement celui des compositions destinées à être appliquées en surface d'un support souple ou massique en vue d'y figurer une couche de protection et/ou de figuration et/ou esthétique et/ou un motif.

Dans les sociétés modernes, une quantité de plus en plus importante de matériaux ou objets entrant dans le champ d'application de l'invention est dédiée à être manipulée quotidiennement et fréquemment par un grand nombre de personnes.

A titre illustratif et non limitatif de ces objets, peuvent notamment être cités les substrats, notamment des billets de banque ou des cartes telles que des cartes à puce, ou objets plastiques tels que par exemple les jouets, les claviers et souris d'ordinateurs, les écrans tactiles et les claviers, écrans et combinés de téléphones, les instruments de soins, médicaux ou de santé, les instruments de musique, les vêtements de travail, les outils, les tissus d'ameublement.

Pour des raisons évidentes, les utilisateurs de ces objets peuvent être porteurs de virus, susceptibles de générer des maladies épidémiques et pandémiques plus ou moins graves et, à ce titre, être susceptibles de contaminer tout objet à leur contact. Or, lorsque cet objet est dédié à être consécutivement manipulé par un ou plusieurs autres utilisateurs, il devient à son tour un important véhicule de dissémination, à l'égard d'autres personnes, du virus véhiculé par le premier utilisateur.

En conséquence, il serait avantageux de pouvoir neutraliser dans un bref délai tout virus en contact avec un objet ou substrat dédié à un usage multiple.

Pour des raisons manifestes, ce mode de neutralisation doit d'une part être efficace et d'autre part avoir une durée prolongée dans le temps. En outre, il doit être facile de mise en oeuvre et dans la mesure du possible ne pas affecter l'usage de l'objet considéré.

Le document US 5 968 538 propose une méthode pour procurer des propriétés anti-pathogéniques à un substrat *via* la formation d'un revêtement à partir d'une composition contenant un agent anti-pathogénique consistant essentiellement en de la povidone iodée (PVP-1) et du nonoxynol (N-9).

La publication Clarke et al., Journal of Medical Microbiology, vol. 49, pages 719-723, rapporte une étude de l'efficacité antivirale de facteurs antimicrobiens présents dans le lait humain contre trois rhinovirus et un isolat de cytomégalovirus (CMV), et indique l'efficacité de la vitamine A, de la monolaurine et de la lactoferrine pour inhiber la croissance du CMV.

La publication Thormar et al., Antimicrobial Agents and Chemotherapy, 1987-01-01, pages 27-31, rapporte une étude de l'efficacité d'un certain nombre d'acides gras, notamment de l'acide laurique et de la monolaurine, contre des virus enveloppés, tels que VSV, VV et HSV-1.

Le document US 4,662,403 propose la mise en oeuvre d'une émulsion de monolaurine pour traiter des emballages en cellulose utilisés pour les saucisses, la monolaurine étant fixée à la surface cellulosique des emballages formés.

Contre toute attente, les inventeurs ont constaté que les objectifs précités sont satisfaits via la mise en oeuvre d'une composition fluide antivirale apte à former un revêtement.

Ainsi, l'invention concerne, selon un de ses aspects, une composition fluide selon la revendication 1.

Au sens de l'invention, une température ambiante s'entend comme une température variant de 18 à 25 °C.

L'invention concerne en particulier une composition fluide apte à former un revêtement en surface de matériaux ou un substrat, et plus particulièrement destinée à recouvrir les objets susceptibles de véhiculer des virus, notamment des jouets pour enfants, ou encore des instruments de soins, médicaux ou de santé, des billets de banque ou des cartes telles que des cartes à puce.

Ainsi, selon un autre de ses aspects, l'invention concerne des objets, notamment tels que décrits ci-dessus, caractérisés en ce qu'il sont susceptibles d'être obtenus par un procédé comprenant au moins une étape de revêtement en surface par une composition fluide selon l'invention.

Selon un de ses aspects, l'étape de revêtement décrite ci-dessus est effectuée par pulvérisation, impression, surimpression, surfaçage, couchage ou dépôt de la composition selon l'invention sur la surface.

D'une manière générale, le virucide requis selon l'invention est formulé dans un milieu solvant, notamment tel que défini ci-après.

Comme il ressort des exemples ci-après, le virucide considéré selon l'invention, s'avère avantageux au-delà de son activité biologique.

Tout d'abord, il se prête à une solubilisation dans les milieux solvants classiquement considérés pour le traitement de surface de support, et qui sont très souvent des solvants aqueux ou des résines à réticulation UV.

Par ailleurs, les solutions correspondantes et en particulier les solutions aqueuses ou à réticulation UV conservent leur aspect coloriel d'origine. En d'autres termes, si le milieu solvant est nativement incolore, ce même milieu formulé avec le virucide conserve cette transparence.

Aussi, dans le cas d'un vernis, le revêtement qu'il forme en surface d'un support, est à la fois efficace et peut être totalement transparent, sous réserve de la nature du milieu solvant retenu.

Cette pseudo invisibilité du revêtement est pour des raisons évidentes, particulièrement intéressante. En particulier, les vernis selon l'invention s'avèrent particulièrement avantageux pour constituer des vernis de surimpression de support d'information tels que des billets. Ils ne gênent pas la visibilité des éléments de sécurité intégrés dans ces billets.

De même, dans le cas d'une encre, peinture ou laque, la présence d'un virucide selon l'invention, ne s'avère pas préjudiciable à l'effet coloriel recherché parallèlement avec le ou les pigments associés.

Par ailleurs, les inventeurs ont constaté que l'activité virucide des revêtements formés selon l'invention en surface de support ne s'avère pas altérée par une exposition prolongée à la lumière ou à une lumière UV.

Enfin, comme détaillé ci-après, l'efficacité virucide, est obtenue avec des concentrations réduites en virucide. De façon surprenante, une quantité inférieure à 3 % voire à 2 % en poids de virucide exprimée en poids sec du revêtement le contenant, en particulier un vernis, s'avère particulièrement efficace.

Selon une variante particulière, les compositions selon l'invention contiennent également en outre au moins un agent humectant.

Les compositions considérées selon l'invention sont plus particulièrement des vernis, des encres, des laques ou des peintures.

Selon un mode de réalisation, les compositions considérées selon l'invention sont des vernis, et plus particulièrement des vernis de surimpression.

En conséquence, les compositions selon l'invention peuvent contenir, outre le virucide, au moins un des composants classiquement considérés dans ce type de formulations.

Ainsi, les agents liants sont des composés conventionnellement utilisés dans les compositions de type vernis et/ou encre. Ils y ont généralement pour fonction d'assurer la dispersion des particules tels que les pigments si présents au sein de la composition et de contribuer, après séchage et/ou réticulation de la composition appliquée en surface d'un support, à la formation d'un film de dureté suffisante pour assurer à ce dernier une pérennité.

Contre toute attente, les inventeurs ont en effet constaté qu'il est possible de concilier la présence au sein d'une même composition de deux types de composés aussi distincts qu'un agent liant et un virucide sans porter préjudice à leurs efficacités respectives.

Les compositions selon l'invention contiennent donc avantageusement en outre au moins un agent liant et le cas échéant au moins un pigment.

L'invention concerne également, selon un autre de ses aspects, un procédé utile pour conférer des propriétés virucides à tout ou partie de la surface d'un support souple ou massique selon la revendication 11.

D'une manière générale, le revêtement attendu est obtenu à l'issue du séchage de la composition appliquée.

Selon une variante de réalisation, le séchage de la composition appliquée est un séchage UV. Selon ce mode de réalisation, les encres ou les vernis obtenus seront qualifiés de vernis « UV » ou d'encre « UV ».

Le virucide selon l'invention est un virucide d'origine naturelle, notamment tel que défini ci-après.

Selon une variante de réalisation, le virucide peut être généré *in situ* à partir d'une composition selon l'invention contenant, à titre d'actif, une forme précurseur de ce virucide.

Ainsi, l'invention concerne également un procédé selon la revendication 12.

## Composition fluide

Comme il ressort de qui précède, une composition fluide selon l'invention présente une viscosité comprise entre 30 mPa.s et 40 Pa.s, en particulier entre 50 mPa.s et 25 Pa.s, mesurée à température et pression ambiante.

Les viscosités des compositions peuvent être mesurées par des méthodes conventionnelles. La sélection de la méthode de mesure de même que l'appareillage de mesure adéquats notamment au regard de l'échelle de viscosité de la composition considérée relève clairement des compétences de l'homme de l'art.

Par exemple, pour une composition possédant une viscosité manifestement inférieure à 2 Pa.s, il est privilégié comme appareillage de mesure un viscosimètre Brookfield avec l'axe n°2 à 100 tours par minute (ISO 2555).

Cette viscosité peut être ajustée au regard de la fonction particulière attachée à la composition à savoir constituer par exemple un vernis, une encre, une laque ou une peinture, mais également au mode d'application considéré pour traiter la surface d'un support avec ladite composition.

Par exemple, une composition fluide selon l'invention peut être déposée en surface d'un support par impression offset, par héliogravure, par flexographie, par surimpression flexographique, par gravure en taille-douce, par typographie ou par lithographie.

Ainsi, à température et pression ambiante, une composition fluide selon l'invention utilisée :

- en héliogravure peut avantageusement présenter une viscosité comprise entre 30 et 50 mPa.s,
- en flexographie peut avantageusement présenter une viscosité comprise entre 30 et 90 mPa.s,
- en surimpression flexographique peut avantageusement présenter une viscosité comprise entre 30 et 50 mPa.s,
- en gravure en taille-douce peut avantageusement présenter une viscosité comprise entre 9 et 25 Pa.s,
- en impression offset peut avantageusement présenter une viscosité comprise entre 2 et 40 Pa.s, et
- en lithographie peut avantageusement présenter une viscosité comprise entre 10 et 20 Pa.s.

La viscosité de la composition fluide selon l'invention peut être ajustée via la nature et/ou quantité du milieu solvant associé au virucide requis selon l'invention, ou encore via l'ajout et l'ajustement de la quantité de liant(s) selon l'invention et dans lequel est formulé le virucide ou précurseur de virucide requis selon l'invention.

**Virucide**

La composition fluide apte à former un revêtement conforme à l'invention contient au moins un virucide et/ou de ses précurseurs.

Au sens de la présente invention, le terme « virucide » désigne tout composé possédant la capacité de tuer ou d'inhiber les virus.

Le virucide selon la présente invention est plus particulièrement dédié à tuer et/ou à inhiber un virus pathogène à l'égard des mammifères et plus particulièrement de l'Homme. De tels virus peuvent être des virus nus ou des virus enveloppés.

A titre représentatif des virus pathogènes pour l'Homme susceptibles d'être considérés selon l'invention, on peut plus particulièrement citer les rétrovirus, les cytomégalovirus, les rotavirus, les paramyxovirus, les poliovirus, les hantavirus, les virus coxsackie, le virus de l'encéphalomyocardite, les picornavirus dont les rhinovirus, les virus à ADN ou à ARN notamment les flaviviridae, le virus du SIDA, les virus de la grippe, le virus de la variole, le virus de la fièvre jaune, le virus de l'hépatite C, les virus de l'herpès, le virus d'Epstein-Barr, le virus varicelle-zona, le virus de la rubéole, ou encore le virus simien 40 ou SV40.

Les virucides peuvent être synthétiques ou d'origine naturelle.

A titre illustratif des virucides synthétiques peuvent notamment être cités les dérivés chlorés et les aldéhydes. Il peut s'agir plus particulièrement de glutaraldehyde, peroxomonosulfate de potassium, perborate de sodium, peroxodisulfate de potassium, et de percarbonate de sodium.

Selon l'invention, le virucide est d'origine naturelle.

Par « virucide d'origine naturelle », on entend désigner tout virucide pré-existant dans la nature ou pouvant être synthétisé à partir de composés naturels existant dans la nature.

Les virucides d'origine naturelle peuvent ainsi être obtenus soit par extraction et purification à partir d'un milieu naturel les contenant, soit par synthèse à partir de composés naturels.

A titre d'exemple de tels virucides, on peut notamment citer l'acide laurique ou la monolaurine qui peut être obtenue par synthèse à partir de glycérol et d'acide laurique.

Dans le cas de cette seconde alternative, le glycérol et l'acide laurique constituent au sens de l'invention un précurseur de virucide dans la mesure où ils permettent, à l'issue du procédé selon l'invention de générer un support souple ou massique à propriétés antivirales.

Plus précisément, le terme « précurseur » désigne, selon l'invention, un composé qui est à même, lors des étapes du procédé d'application selon l'invention, soit par transformation soit par réaction avec un autre composé qui lui est associé, et donc également qualifié de précurseur, de générer le virucide attendu.

On peut décrire comme virucides l'acide laurique, la monolaurine, la lactoferrine et les huiles essentielles présentant une activité antivirale, comme par exemple l'huile essentielle de laurier. Selon l'invention le virucide contient au moins la monolaurine. Au sens de l'invention, le terme monolaurine entend désigner à la fois la monolaurine pré-existant naturellement et celle obtenue par synthèse à partir de glycérol et d'acide laurique.

Ce type de virucide d'origine naturelle a en effet été identifié comme présentant des propriétés particulièrement avantageuses pour la préparation de compositions fluides aptes à former un revêtement telles que considérées dans le cadre de la présente invention.

Dans le cadre de la présente invention, préférentiellement, la synthèse de la monolaurine à partir de l'acide laurique est effectuée à une température d'environ 100 °C, de préférence supérieure ou égale à 100 °C, de sorte qu'elle peut notamment être réalisée lors de la préparation de vernis dans les fours ou encore lors de la réticulation ou du séchage d'encres.

La composition fluide apte à former un revêtement conforme à l'invention contient une quantité efficace d'au moins un virucide et/ou au moins un de ses précurseurs, c'est-à-dire une quantité suffisante de celui-ci pour doter la composition l'incorporant de propriétés antivirales.

Selon un mode de réalisation, il peut notamment s'agir d'une quantité suffisante de virucide pour conférer à ladite composition l'incorporant une activité antivirale supérieure à 1 log, suivant le protocole de mesure décrit dans les exemples.

Pour des raisons évidentes, la quantité de virucide à mettre en oeuvre selon l'invention dépend notamment de la nature dudit virucide et/ou de la nature de ladite composition et peut donc varier dans une large mesure.

L'homme du métier peut aisément, sur la base de ses connaissances générales, déterminer les quantités appropriées. L'ajustement de la quantité de virucide fait partie des compétences de l'homme du métier.

Les inventeurs ont notamment déterminé qu'une quantité de virucide inférieure à 2 % en poids permet d'obtenir une activité antivirale satisfaisante.

Une composition fluide conforme à l'invention contient de 0,1 % à 3 % en poids sec, par exemple de 0,1 % à 2 % en poids sec, par exemple de 0,5 % à 1,5 % en poids sec, de virucide par rapport à son poids total.

Selon un mode de réalisation, la composition fluide conforme à l'invention peut contenir en outre d'autres composés actifs additionnels, présentant ou non une activité antivirale.

Il peut notamment contenir en outre des biocides, et par exemple des biocides de type bactériostatique et/ou bactéricide et/ou fongistatique et/ou fongicide.

Ainsi, selon un mode de réalisation, la composition selon l'invention contient outre le virucide requis, au moins un bactéricide et/ou un fongicide.

A titre illustratif des bactéricides peuvent notamment être cités les sels d'argent réfractaires, les sels d'ammonium quaternaire tels que le chlorure de myristyl dimethyl benzyl ammonium ou le saccharinate d'alkyl diméthyl benzyl ammonium, les pyrithiones et ses dérivés.

De préférence, la composition ne comprend pas de bactéricide toxique. En particulier, elle est exempte de pyrithiones et de ses dérivés.

A titre illustratif des fongicides peuvent notamment être cités le diiodométhyl-p-tolylsulfone ou le 3-iodopropargyl-N-butylcarbamate.

De préférence, la composition ne comprend pas de fongicide toxique. En particulier, elle est exempte de diiodométhyl-p-tolylsulfone.

Selon un autre mode de réalisation, le virucide requis selon l'invention peut présenter lui-même, outre son activité antivirale, au moins une autre activité biologique.

Ainsi, le virucide requis selon l'invention peut par exemple présenter en outre une activité bactériostatique, bactéricide, fongistatique ou fongicide, et plus particulièrement une activité bactériostatique ou bactéricide.

Comme précisé précédemment, le virucide peut être mis en oeuvre sous une forme associée à un agent humectant.


**Agent humectant**

Au sens de l'invention, un agent humectant est un composé apte à procurer un effet d'hydratation ou encore hygroscopique.

Contre toute attente, les inventeurs ont constaté que la présence d'un tel composé peut permettre de stimuler l'activité antivirale du virucide, en particulier d'un virucide d'origine naturelle associé, et donc d'accroître l'activité antivirale manifestée par une composition fluide conforme à l'invention incorporant ces deux composés.

A titre représentatif de ces agents humectants, peuvent être tout particulièrement considérés, dans le cadre de la présente invention, les composés de type polyol, tels que, par exemple, la glycérine, dite encore glycérol, le propylène glycol, le polyéthylène glycol, le butylène glycol, le triacétate de glycéryle, ou encore le sorbitol.

Selon une variante de réalisation préférée, l'agent humectant considéré est le glycérol.

Selon une autre variante de réalisation, l'agent humectant considéré est choisi parmi les composés suivants :

- l'acide pidolique (PCA) et ses dérivés (arginine PCA, copper PCA, ethylhexyl PCA, lauryl PCA, magnesium PCA, sodium PCA, zinc PCA...),
- le gluconate de calcium,
- le fructose, le glucose, l'isomalt, le lactose, le maltitol, le mannitol, le polydextrose, le sorbitol, le saccharose ou le xylitol,
- l'acide glycyrrhizique et ses dérivés,
- l'histidine,
- l'acide hyaluronique et ses sels tels que l'hyaluronate de sodium,
- les hydrolysats de soie, de kératine ou de soja,
- le phytantriol,
- la soie, ou
- l'urée.

La composition fluide conforme à l'invention peut contenir de 0,5 % à 4 % en poids sec, par exemple de 1 % à 3 % en poids sec d'agent(s) humectant(s), et notamment de glycérol, par rapport à son poids total.

Selon un mode de réalisation préféré, l'agent humectant est présent dans la composition fluide apte à former un revêtement conforme à l'invention en un rapport pondéral masse d'agent(s) humectant(s) sur masse de virucide(s) au moins égal à 1.

Selon un mode de réalisation particulier, la composition fluide conforme à l'invention peut contenir au moins un virucide selon la présente invention, au moins un agent humectant, notamment du glycérol, et en outre au moins un biocide bactériostatique et/ou bactéricide ou un biocide fongistatique et/ou fongicide.

Selon un autre mode de réalisation particulier, la composition fluide conforme à l'invention peut contenir au moins un précurseur du virucide selon la présente invention, au moins un agent humectant, notamment du glycérol, et en outre au moins un biocide bactériostatique et/ou bactéricide et au moins un biocide fongistatique et/ou fongicide.

Le virucide et/ou un de ses précurseurs et l'agent humectant si présent sont formulés avantageusement dans un milieu solvant.

## Milieu solvant

La nature de ce milieu solvant est directement liée au type de composition visée.

Le milieu solvant peut être monophasique ou biphasique.

Par exemple, un milieu solvant selon l'invention peut se présenter sous la forme d'une émulsion huile dans eau ou eau dans huile.

Comme précisé précédemment, les compositions selon l'invention sont plus particulièrement des encres, des vernis, des laques ou des peintures.

Plus préférentiellement, les compositions selon l'invention sont des encres ou des vernis.

D'une manière générale, l'ensemble de ces compositions mettent en oeuvre à titre de milieu solvant de l'eau, un solvant organique, une huile ou un de leurs mélanges.

Par exemple, parmi les encres, il existe des encres aqueuses dont le solvant est l'eau, ainsi que des " encres solvantées " dont le solvant est organique, et des encres grasses. Par encres grasses, on entend les différentes encres utilisées pour l'impression typographique, la lithographique ou l'impression des gravures en taille-douce.

Ainsi, une composition fluide selon l'invention peut comprendre au moins un milieu solvant organique composé d'au moins un solvant organique volatil à température ambiante.

Comme solvant organique volatil ou non à température ambiante, on peut citer :

- les cétones liquides à température ambiante tels que la méthyléthylcétone, la méthylisobutylcétone, la diisobutylcétone, l'isophorone, la cyclohexanone, acétone ;
- les alcools liquides à température ambiante tels que l'éthanol, l'isopropanol, le butanol, le diacétone alcool, le 2-butoxyéthanol, le cyclohexanol, le 2-amino-2-méthyl-1-propanol ;
- les glycols liquides à température ambiante tels que l'éthylène glycol, le propylène glycol, le pentylène glycol, le glycérol, le 2-amino-2-ethyl-1,3-propane-diol ;
- les éthers de propylène glycol liquides à température ambiante tels que le monométhyléther de propylène glycol, l'acétate de monométhyl éther de propylène glycol, le mono n-butyl éther de dipropylène glycol ;
- les esters à chaînes courtes (ayant de 3 à 8 atomes de carbone au total) tels que l'acétate d'éthyle, l'acétate de méthyle, l'acétate de propyle, l'acétate de butyle, l'acétate d'aryle, l'acétate d'isopentyle ;
- les alcanes liquides à température ambiante tels que le décane, l'heptane, l'octane, le dodécane, le cyclohexane, l'isododécane ; et
- leurs mélanges.

En ce qui concerne les huiles, elles peuvent être choisies parmi :

- les huiles végétales ;
- l'huile de lin purifiée ;
- l'huile de tung, notamment pour des encres à séchage rapide ;
- l'huile de soja, notamment dans le domaine de l'imprimerie. Cette huile est appréciée pour l'amélioration qu'elle apporte au mouillage des pigments ;
- l'huile de tournesol, notamment pour préparer des résines alkydes ;
- l'huile de colza ;
- le tall oil ; et
- les distillats pétroliers.

Les compositions selon l'invention peuvent également mettre en oeuvre à titre de milieu solvant une résine réticulable UV.

Par exemple, il existe des encres flexographiques UV mettant en oeuvre ce type de résine à titre de milieu solvant.

Outre le virucide ou précurseur de virucide, une composition selon l'invention contient les composants conventionnellement mis en oeuvre dans ce type de composition.

Ainsi, une encre, un vernis, une laque ou une peinture est généralement composé d'un ou plusieurs pigments et d'un agent liant.

**Liants**

Comme précisé ci-dessus, une composition fluide apte à former un revêtement selon l'invention contient en outre au moins un agent liant.

Plus particulièrement, un agent liant selon l'invention est choisi parmi les résines, les cires, et les gommes.

Une résine peut être notamment choisie parmi les résines époxy cycloaliphatiques, acryliques, vinyliques, cétoniques, polyesters, et aldéhydes.

Une résine peut être présente dans une composition selon l'invention dans une teneur comprise entre 15 % et 60 % en poids, de préférence entre 20 % et 40 % en poids, par rapport au poids total de la composition.

A titre de cires pouvant être utilisées selon l'invention, on peut citer :

- les cires végétales telles que la cire de Carnauba, de Candellila, d'Ouricury, du Japon, le beurre de cacao ou les cires de fibres de liège ou de canne à sucre,
- les cires minérales, par exemple de paraffine, de vaseline, de lignite ou les cires microcristallines ou les ozokérites,
- les cires synthétiques parmi lesquelles les cires polyoléfiniques notamment de polyéthylène, et les cires obtenues par synthèse de Fisher-Tropsch,
- les cires de silicone, en particulier les polysiloxanes linéaires substitués; on peut citer, par exemple, les cires de silicone polyéther, les alkyl ou alkoxy-diméthicones ayant de 16 à 45 atomes de carbone, les alkyl méthicones comme la $C_{30}$-$C_{45}$ alkyl méthicone vendue sous la dénomination commerciale « AMS C 30 » par DOW CORNING,
- les huiles hydrogénées,
- et/ou leurs mélanges.

A titre illustratif des cires convenant à l'invention, on peut notamment citer les cires hydrocarbonées comme la cire d'abeilles, la cire de lanoline, et les cires d'insectes de Chine; la cire de son de riz, la cire de Carnauba, la cire de Candellila, la cire d'Ouricury, la cire d'Alfa, la cire de berry, la cire de shellac, la cire du Japon et la cire de sumac; la cire de montan, les cires d'orange et de citron, les cires micro cristallines, les paraffines et l'ozokérite; les cires de polyéthylène, les cires obtenues par la synthèse de Fisher-Tropsch et les copolymères cireux ainsi que leurs esters.

Une cire peut être présente dans une composition selon l'invention dans une teneur comprise entre 0 % et 20 % en poids, de préférence entre 0,5 % et 15 % en poids, par rapport au poids total de la composition.

Une gomme peut être notamment choisie parmi les gommes arabiques, les gommes adragantes, les gommes de casse, les gommes de gutte, les gommes de laque, les sandaraques, les gommes de mastic ou les gommes-résines.

La nature du liant conditionne généralement le type de vernis à savoir vernis cellulosiques, vernis polyuréthanes, ou encore vernis acryliques.

De préférence, une composition fluide selon l'invention est une encre et elle contient au moins un pigment et au moins un agent liant en particulier choisi parmi une cire polyéthylénique, une résine acrylique et leurs mélanges.

De préférence, une composition fluide selon l'invention est un vernis et elle contient au moins un agent liant en particulier choisi parmi une cire polyéthylénique, une résine acrylique et leurs mélanges.

De préférence, une composition fluide selon l'invention est un vernis de surimpression et elle contient au moins un agent liant en particulier choisi parmi les résines époxy cycloaliphatiques.

**Pigments**

Une composition fluide apte à former un revêtement selon l'invention peut avantageusement contenir un ou plusieurs pigment(s).

Les pigments peuvent être présents à raison de 0 % à 60 % en poids, notamment de 10 % à 50 % en poids, et en particulier de 15 % à 35 % en poids, par rapport au poids total de la composition fluide apte à former un revêtement selon l'invention.

De préférence, la composition fluide selon l'invention est une encre, et les pigments peuvent être présents à raison de 0 % à 60 % en poids, notamment de 10 % à 50 % en poids, et en particulier de 15 % à 35 % en poids, par rapport au poids total de la composition fluide apte à former un revêtement selon l'invention.

Par pigments, il faut comprendre des particules blanches ou colorées, minérales ou organiques, insolubles dans une solution aqueuse, destinées à colorer et/ou opacifier le film résultant.

Comme pigments minéraux utilisables dans l'invention, on peut citer les oxydes de titane, de zirconium ou de cérium ainsi que les oxydes de zinc, de fer ou de chrome, le bleu ferrique, le violet de manganèse, le bleu outremer et l'hydrate de chrome.

Parmi les pigments organiques utilisables dans l'invention, on peut citer le noir de carbone, les pigments de type D & C, les laques à base de carmin de cochenille, de baryum, strontium, calcium ou aluminium.

Selon une variante de réalisation, ces pigments peuvent être également des pigments perlescents ou dits encore nacrés, et/ou des pigments luminescents et en particulier fluorescents ou phosphorescents.

Comme pigments nacrés utilisables dans l'invention, on peut citer le mica titane recouvert avec un oxyde de fer, le mica titane recouvert d'oxychlorure de bismuth, le mica titane recouvert avec de l'oxyde de chrome, le mica titane recouvert avec un colorant organique ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques.

On peut également citer, à titre d'exemple de pigments nacrés, le mica naturel recouvert d'oxyde de titane, d'oxyde de fer, de pigment naturel ou d'oxychlorure de bismuth.

Comme substances fluorescentes inorganiques utilisables dans la présente invention, on peut par exemple citer les substances fluorescentes inorganiques à base d'oxyde de zinc, les pigments fluorescents à la lumière du jour qui sont généralement fabriqués à partir de colorants fluorescents qui sont préalablement dissous dans une résine support afin d'obtenir une solution solide laquelle est ensuite broyée en une poudre de particules de résine présentant des propriétés fluorescentes.

Les pigments fluorescents convenant à la présente invention peuvent être choisis parmi les résines colorées de polyamide et/ou de formaldéhyde/benzoguanamine et/ou de melamine/formaldéhyde/ sulfonamide, parmi les co-condensats aminotriazine/formaldéhyde/sulfonamide colorés et/ou parmi les paillettes polyester métallisées et/ou leurs mélanges. Ces pigments fluorescents peuvent aussi se présenter sous la forme de dispersions aqueuses de pigments fluorescents.

Lorsque les substances fluorescentes organiques sont blanches, on les appelle également des azurants optiques absorbant essentiellement dans l'UVA entre 300 et 390 nm et remettant essentiellement entre 400 et 525 nm.

Les compositions peuvent également comprendre un ou plusieurs additifs permettant d'optimiser les caractéristiques du revêtement pendant et après son application.

Parmi les additifs, on trouve notamment des agents dispersants, des agents anti-mousses, mais également des polymères, des épaississants et des plastifiants.

## Procédé d'application

Un autre objet de l'invention concerne un procédé d'application d'une composition fluide apte à former un revêtement telle que définie précédemment.

Selon un premier mode de réalisation, il peut s'agir d'un procédé utile pour conférer des propriétés virucides à tout ou partie de la surface d'un support souple ou massique selon la revendication 11.

Concernant l'incorporation dudit virucide en particulier d'origine naturelle au niveau de ladite composition, on peut être amené à utiliser des émulsions ou des solutions particulières, par exemple telles que des solutions ammoniacales ou de préférence à base de 2-amino-2-méthyl-1-propanol qui présente l'avantage de ne pas engendrer de dégagement odorant.

Selon une variante de réalisation, l'agent humectant peut être présent dans une telle émulsion.

Le virucide en particulier d'origine naturelle peut être tel que défini précédemment, et notamment choisi parmi la monolaurine, la lactoferrine et une huile essentielle présentant une activité antivirale, comme par exemple l'huile essentielle de laurier. Selon l'invention le virucide contient au moins la monolaurine. L'agent humectant peut également être tel que défini précédemment, et notamment être du glycérol.

L'application de ladite composition sur la surface du support souple ou massique à recouvrir peut se faire de diverses manières :

- par pulvérisation de ladite composition sur la surface,
- par impression de ladite composition sur la surface,
- par surimpression de ladite composition sur la surface au moins partiellement imprimé,
- par surfaçage de ladite composition sur la surface,
- par couchage de ladite composition sur la surface, et
- par dépôt de ladite composition sur la surface.

L'agent humectant est avantageusement présent dans la composition fluide selon l'invention.

En particulier, ladite application de la composition fluide selon l'invention peut être favorisée par l'utilisation d'une émulsion de monolaurine.

Selon une variante de réalisation, le virucide peut être généré *in situ* à partir d'une composition selon l'invention contenant à titre d'actif une forme précurseur de ce virucide.

Ainsi, selon un autre de ses aspects, l'invention concerne également un procédé selon la revendication 12.

Selon une variante de réalisation, ce procédé peut comprendre en outre la mise en oeuvre d'un agent humectant, notamment tel que défini ci-dessus.

Cette variante de réalisation est particulièrement adaptée lorsque le virucide est d'origine naturelle et que celui-ci est par exemple aisément accessible par synthèse, de préférence à des coûts en outre avantageux.

Ainsi, il pourra s'agir par exemple de la monolaurine synthétisée *in situ* par réaction d'acide laurique et de glycérol en présence d'un catalyseur.

La monolaurine est en effet disponible par ailleurs commercialement mais à des prix relativement élevés. Sa synthèse *in situ* selon cette variante de réalisation permet donc de la mettre en oeuvre dans une composition fluide de type revêtement à un coût réduit.

Concernant l'incorporation dudit virucide au niveau de ladite composition, elle peut être favorisée par l'utilisation d'une solution d'acide laurique, notamment telle qu'une solution ammoniacale ou de préférence à base de 2-amino-2-méthyl-1-propanol qui présente l'avantage de ne pas engendrer de dégagement odorant.

Selon ce deuxième mode de réalisation, le procédé peut comprendre au moins les étapes consistant à :

a) Disposer d'un support souple ou massique dont la surface à traiter comporte au moins un catalyseur et/ou réactif apte à stimuler l'interaction entre l'acide laurique et le glycérol ;
b) Mettre en contact ladite surface avec une composition fluide telle que définie ci-dessus contenant au moins de l'acide laurique et du glycérol ; et
c) Imposer à la surface traitée en étape b) un traitement thermique propice à la synthèse de la monolaurine ;

lesdites étapes b) et c) pouvant être réalisées consécutivement ou simultanément.

Selon une autre variante de réalisation, le catalyseur peut être présent dans la composition fluide contenant l'acide laurique et le glycérol.

Selon une variante de réalisation, ce procédé peut être réalisé en présence d'un agent anti-mousse.

Plus particulièrement, il s'agit d'un composé commercialisé sous le nom Aerotech 3514® (KEMIRA CHIMIE SA) et qui est formé d'un mélange d'huiles minérales et de tensioactifs non ioniques.

Un tel composé peut être introduit à une concentration comprise entre 0,01 % et 0,30 %, de préférence entre 0,04 % et 0,20 %, et plus préférentiellement entre 0,04 % et 0,12 % par rapport au poids total du mélange d'acide laurique et de glycérol.

Comme indiqué précédemment, la synthèse de monolaurine à partir d'acide laurique et de glycérol se déroule en présence d'un catalyseur.

A titre d'exemple de catalyseur convenant plus particulièrement à la catalyse de cette réaction, on peut notamment citer les zéolites, et par exemple la zéolite A commercialisée par la société FMC Foret ou les lipases.

Dans le cas où le catalyseur est une lipase, on peut notamment se référer aux conditions de réaction décrites par Pereira C.C.B., Da Silva M.A.P. et Langone M.A.P. dans la publication « Enzymatic synthesis of monolaurine » (Applied biochemistry and Biotechnology, 2004, vol. 113-116, P.433-445).

A titre de lipase convenant plus particulièrement dans le cadre de la présente invention, on peut par exemple citer les lipases commercialisées sous les références Lipozyme RM IM®, Lipozyme TL IM® et Resinase A2C® par la société NOVOZYMES.

La composition fluide conforme à l'invention peut contenir de 0,5 % à 3 % en poids sec, par exemple de 0,5 % à 2 % en poids sec, de catalyseur par rapport à son poids total.

Le catalyseur, par exemple la zéolite, peut être introduit à raison d'au moins 2 % en poids, par exemple au moins 5 % en poids, par rapport au poids total du mélange d'acide laurique et de glycérol.

Selon une première variante de réalisation, l'acide laurique et le glycérol peuvent être introduits en mélange équimolaire.

Selon une deuxième variante de réalisation, le glycérol peut être introduit en excès par rapport à l'acide laurique.

Selon cette deuxième variante, du glycérol en excès résiduel reste donc présent dans le revêtement à la fin de la réaction.

Comme mentionné précédemment, ce glycérol résiduel peut jouer le rôle d'agent humectant et augmenter les propriétés antivirales.

Les exemples suivants, non limitatifs, permettront de mieux comprendre comment l'invention peut être mise en pratique et ses avantages.

**Exemple 1**

Préparation d'une émulsion de monolaurine

On agite au Rayneri sous bain-marie à 50 °C, 50 g de monolaurine de synthèse jusqu'à ce qu'elle soit fondue.

On ajoute 5g de Disponil TD® 0785 et 7 g d'eau.

On ajoute 4 g d'Eumulgin BA® 10 fondu à 50 °C puis après homogénéisation, on laisse refroidir à température ambiante sous agitation.

On ajoute 6 g d'eau sous agitation puis on laisse agiter pendant 15 mn.

On introduit lentement 47,5g d'eau sous agitation.

On obtient une émulsion de monolaurine à 42 %.

Encre flexographique

Tableau I

|  | % | % sec |
|---|---|---|
| Slurry Kaolin : Intrafill 60 (ES 60 %) | 33.4 | 20 |
| Joncryl 1674 (ES 41%) | 56.8 | 23.3 |
| PE Wax Emulsion : Aquacer 2500 (ES 40 %) | 4.8 | 1.9 |
| Antimousse Nopco 8034 | 0.48 | |
| Emulsion de monolaurine à 42 % selon l'exemple 1 | 4.5 soit 1.9 % de monolaurine | 1.9 |
| Total | 100 % | 47.1 % |

La préparation est faite au Rayneri.

On couche l'encre flexographique préparée comme indiquée ci-dessus sur deux faces d'un support papier classique (velin NS 2005 5175).

On sèche chaque face pendant 3 mn à 100 °C.

La dépose moyenne par face est de 14.4 $g/m^2$ humide soit environ 6.8 $g/m^2$ sec (0.27 $g/m^2$ de monolaurine).

Vernis de sur-impression

Tableau II

|  | % | % sec |
|---|---|---|
| Joncryl 1674 (ES 41 %) | 62.0 | 26.7 |
| Joncryl 8078 (ES 32 %) | 19.1 | 6.1 |
| PE Wax Emulsion :Aquacer 2500 (ES 40 %) | 6.7 | 2.7 |
| eau | 7.6 | |
| Emulsion de monolaurine à 42 % | 4.5 soit 1.9 % de monolaurine | 1.9 |
| Total | 100 | 37.4 |

La préparation est faite au Rayneri.

On couche le vernis de sur-impression préparé comme indiqué ci-dessus sur deux faces d'un support plastique Polyart® (Polyart P3 non couché).

On sèche chaque face pendant 2 mn à 90 °C.

La dépose moyenne par face est de 15.8 $g/m^2$ humide soit environ 5.8 $g/m^2$ sec (0.30 $g/m^2$ de monolaurine).

**Exemple 2**

Activité antiphagique de support traité selon l'invention

Un test de l'activité antiphagique est réalisé. Le test est basé sur la norme JIS L 1902 modifiée, ou encore sur la norme ISO 20743 modifiée, sur les phages MS2, qui sont réputés être très résistants, et appliquée sur des temps d'action compris entre 18 et 24 heures.

Le principe est le suivant : des phages MS2 sont déposés sur le support papier classique (velin NS 2005 5175) considéré en 1$^{ère}$ partie de l'exemple 1 , puis le nombre de phages MS2 actifs est évalué une première fois à t=0h, et une deuxième fois à t=24h.

Pour évaluer le nombre de phages MS2 actifs sur les supports à tester à un temps donné, on met ces supports en présence de bactéries particulières qui présentent la propriété d'être des hôtes des phages MS2 : la mesure du nombre de plages de lyses (ou pfp) après culture permet alors de remonter à la quantité de phages MS2 recherchée.

On peut ainsi en déduire une activité antiphagique (notée A), définie comme suit :

$$A = [moy \log (C_{24}) - moy \log (C_0)] - [moy \log (E_{24}) - moy \log (E_0)],$$

formule dans laquelle $E_{24}$ correspond au nombre de plages de lyses à 24h et $E_o$ correspond au nombre de plages de lyses juste après sa mise en contact avec le support testé.

Les conditions expérimentales sont les suivantes :

- Le diluant utilisé est le peptone/sel (de référence DIFCO, 1897-17) et la souche bactérienne utilisée est *Escherichia coli* K12, qui est une souche hôte des phages MS2.
- Le support témoin est un textile 100 % coton non traité.
- On dépose 200 μL d'une suspension de phages à 1 x 10$^5$ pfp/mL.

On en déduit l'activité antiphagique suivante :

$$A_{encre\ flexographique\ selon\ l'exemple\ 1} = - 2,74 - (-3,94) = 1,20\ \log$$

Les résultats sont reportés ci-après.

Tableau III

| Temps d'incubation | | 0h | | | | 24h | | | |
|---|---|---|---|---|---|---|---|---|---|
| Echantillon | éprouvette | Co (pfp/ech) | log (Co) | moy Co | log (moy Co) | $C_{24}$ (pfp/ech) | log ($C_{24}$) | moy $C_{24}$ | log (moy $C_{24}$) |
| Témoin = Support textile sans encre | 1 | 6 300 000 | 6,80 | 5580 000 | 6,75 | 4300 | 3,63 | 10150 | 4,01 |
| | 2 | 4 860 000 | 6,69 | | | 16000 | 4,20 | | |

| Temps d'incubation | | 0 h | | | | 24 h | | | |
|---|---|---|---|---|---|---|---|---|---|
| Echantillon | éprouvette | Eo (pfp/ech) | log (Eo) | moy Eo | log (moy Eo) | $E_{24}$ (pfp/ech) | log ($E_{24}$) | moy E24 | log (moy $E_{24}$) |
| **Support traité avec encre flexographique selon l'exemple 1** | 1 | 2 900 000 | 6,46 | 4950 000 | **6,69** | 430 | 2,63 | 560 | **2,75** |
| | 2 | 7 000 000 | 6,85 | | | 690 | 2,84 | | |

**Exemple 3**

Activité antivirale de support traité selon l'invention

Deux tests de l'activité antivirale sont réalisés, l'un au regard de Poliovirus Lsc 1 et l'autre au regard de Influenza A (H1N1).

Le support testé est le support plastique Polyart® (Polyart P3 non couché) considéré en 2ème partie de l'exemple 1.

a) Poliovirus Lsc 1

Le protocole opératoire est comparable au test ASTM E 1053-97 (Standard Test Method for Efficacy of Virucidal Agents Intended for Inanimate Surfaces).

Les supports sont des papiers unis en coton non traités.

Le principe est le suivant :

Les papiers traités et non traités sont coupés en sections de 30 mm$^2$.

Cinq de ces sections traitées et non traitées sont placées dans des boites de Pétri stériles en plastique de 250 mm.

100 microlitres d'une dilution comprenant des virus tels que définis ci-dessus sont appliqués uniformément sur la surface des sections carrées.

Les cinq dilutions testées à la fin sur le support traité selon l'invention et le support témoin sont précisées dans le Tableau IV.

Les boites de Pétri sont couvertes et incubées pendant 24h à 22 °C.

Elles sont ensuite retirées et chaque section inoculée est transférée dans un tube à centrifuge stérile à fond conique (Fisher scientific, PA). Pour chaque tube, on ajoute 20 mL de PBS (Phosphate Buffered saline) stérile et de l'extrait de boeuf à 3 % (Becton Dickinson # 263010, MD).

Les tubes sont placés sur un agitateur orbital et agités à basse vitesse pendant 15 minutes.

Après agitation, on retire 5 ml de liquide de chaque tube que l'on place chacun dans un nouveau tube à centrifuge stérile à fond conique (Fisher scientific, PA).

Les suspensions sont dilués dix fois dans du PBS.

Le nombre de poliovirus viable dans chaque tube est recensé.

Le recensement est effectué par inoculation aliquote des dilutions d'échantillon sur des monocouches de cellules BGM fraîchement préparée en utilisant un revêtement d'agar.

Les plaques sont enregistrées durant une période de 2-4 jours d'incubation.

Les cellules sont incubées à 35 ° C dans une atmosphère à 5 % de $CO_2$.

Les résultats sont reportés ci-après.

Tableau IV

| La concentration initiale d'unités virales infectieuses par échantillon est de 1700 pfu/ml (pfu = plaque forming units) ; Les résultats sont appréciés après un temps de contact de 24h. | | |
|---|---|---|
| | Concentration de Poliovirus pfu/ml % | Pourcentage moyen de réduction de la concentration de virus |
| Support traité avec vernis de sur-impression selon l'exemple 1 | 23 | |
| Support traité avec vernis de sur-impression selon l'exemple 1 | 28,5 | |
| Support traité avec vernis de sur-impression selon l'exemple 1 | 22 | 98,8 |
| Support traité avec vernis de sur-impression selon l'exemple 1 | 17 | |
| Support traité avec vernis de sur-impression selon l'exemple 1 | 10,5 | |

(suite)

| | Concentration de Poliovirus pfu/ml % | Pourcentage moyen de réduction de la concentration de virus |
|---|---|---|
| La concentration initiale d'unités virales infectieuses par échantillon est de 1700 pfu/ml (pfu = plaque forming units) ; Les résultats sont appréciés après un temps de contact de 24h. | | |
| Témoin 1 | 170 | 53,9 |
| Témoin 2 | 650 | |
| Témoin 3 | 900 | |
| Témoin 4 | 110 | |
| Témoin 5 | 1100 | |

b) Influenza A (H1N1)

Préparation des cultures de virus :

Le virus Influenza A (H1N1 ; ATCC VR-1469) est propagé et recensé comme nombres les plus probables (MPN = Most Probable Numbers) en utilisant comme hôtes des monocouches (ATCC CCL-34) de cellules de MDCK (Madin-Darbin Canine Kidney type I).

Les cellules sont cultivées dans des plaques de culture cellulaire à 12 puits.

Pour le recensement, les parties aliquotes d'un échantillon sont inoculées sur des monocouches fraichement préparées de monocouches de MDCK.

Les cellules sont incubées dans un milieu dMEM (MediaTech, USA) contenant de la trypsine, à 35 °C et sous une atmosphère à 5 % en $CO_2$ pendant 5-7 jours.

Les cellules sont surveillées systématiquement au microscope pour observer les signes de dégénérescence.

Les cellules dans les puits présentant des signes d'infectiosités (Cytopathic effects ; CPE) sont enregistrés comme positives (+) et celles qui n'en présentent pas sont enregistrées comme négatives (-).

Le nombre le plus probable de virus infectieux dans un échantillon est ensuite calculé en utilisant un logiciel MPNCALC (version 0.0.0.23).

Pour les expériences, un stock viral congelé (typiquement 1 x $10^8$ iu/ml) est rapidement décongelé dans un bain d'eau à 35 °C le jour précédent les expériences.

On fait ensuite une dilution 1/10 du stock dans du PBS complété avec du BSA (Bovine Serum Albumin) à 2 %.

Le stock est ensuite utilisé pour le test antiviral qui suit.

Le stock de virus dilué est titré par dix dilutions successives de PBS et inoculé sur des cellules de MDCK comme décrites ci-dessus.

Le mode opératoire pour le test antiviral est le même que celui décrit ci-dessus.

Le nombre de Influenza A dans chaque tube est recensé.

Le recensement est effectué selon la procédure MPN décrite ci-dessus.

Les résultats sont reportés ci-après.

Tableau V

| Le MPN calculé initial est de 460000 et le nombre d'unités virales infectieuses inoculées par échantillon est de 46000. Les résultats sont appréciés après un temps de contact de 24h. | | |
|---|---|---|
| | Nombre le plus probable (MPN) calculé de Influenza A % | Pourcentage moyen de réduction de la concentration de virus |
| Support traité avec vernis de sur-impression selon l'exemple 1 | < 0,4 | > 99,999 |
| Support traité avec vernis de sur-impression selon l'exemple 1 | < 0,4 | |
| Support traité avec vernis de sur-impression selon l'exemple 1 | < 0,4 | |
| Support traité avec vernis de sur-impression selon l'exemple 1 | < 0,4 | |
| Support traité avec vernis de sur-impression selon l'exemple 1 | < 0,4 | |
| Témoin 1 | 460 | 98,2 |
| Témoin 2 | 1100 | |
| Témoin 3 | 460 | |
| Témoin 4 | 1100 | |
| Témoin 5 | 1100 | |

**Revendications**

1. Composition fluide apte à former un revêtement **caractérisée en ce qu'**elle contient de 0,1 % à 3 % en poids sec par rapport au poids total de la composition d'au moins un virucide d'origine naturelle, ledit virucide d'origine naturelle étant la monolaurine,
ladite composition possédant une viscosité comprise entre 30 mPa.s et 40 Pa.s, à température et pression ambiante, ladite composition comprenant en outre au moins un agent liant choisi parmi les résines, les cires et les gommes.

2. Composition selon la revendication 1, **caractérisée en ce qu'**elle contient en outre au moins un biocide bactériostatique et/ou bactéricide ou un biocide fongistatique et/ou fongicide.

3. Composition selon l'une quelconque des revendications précédentes, **caractérisée en ce qu'**elle contient de 0,1 % à 2 % en poids sec, par exemple de 0,5 % à 1,5 % en poids sec, dudit virucide par rapport à son poids total.

4. Composition selon l'une quelconque des revendications précédentes, **caractérisée en ce qu'**elle contient en outre un agent humectant, ledit agent humectant étant en particulier un polyol et plus particulièrement du glycérol.

5. Composition selon l'une quelconque des revendications précédentes, **caractérisée en ce que** le rapport pondéral masse d'agent humectant sur masse de virucide est au moins égal à 1.

6. Composition selon l'une quelconque des revendications précédentes, **caractérisée** en qu'elle est de type encre ou vernis.

7. Composition selon l'une quelconque des revendications précédentes, **caractérisée en ce qu'**il s'agit d'une encre contenant au moins un pigment et au moins un agent liant en particulier choisi parmi une cire polyéthylénique, une résine acrylique et leurs mélanges.

8. Composition selon l'une quelconque des revendications précédentes, **caractérisée** en qu'elle contient une quantité de pigments comprise entre 0 % et 60 % en poids, notamment entre 10 % et 50 % en poids, et en particulier comprise entre 15 % et 35 % en poids, par rapport au poids total de ladite composition.

9. Composition selon l'une quelconque des revendications 1 à 6, **caractérisée en ce qu'**il s'agit d'un vernis contenant au moins un agent liant en particulier choisi parmi une cire polyéthylénique, une résine acrylique et leurs mélanges.

10. Composition fluide selon l'une quelconque des revendications 1 à 6, **caractérisée en ce qu'**il s'agit d'un vernis de surimpression contenant au moins un agent liant en particulier choisi parmi les résines époxy cycloaliphatiques.

11. Procédé utile pour conférer des propriétés virucides à tout ou partie de la surface d'un support souple ou massique comprenant au moins l'étape consistant à appliquer une composition telle que définie en revendications 1 à 10, ledit support étant choisi dans le groupe constitué des billets de banque, des cartes telles que des cartes à puce, des objets plastiques tels que les jouets, les claviers et souris d'ordinateurs, des écrans tactiles, des claviers, écrans et combinés de téléphones, des instruments de soins, médicaux ou de santé, des instruments de musique, des vêtements de travail, des outils et des tissus d'ameublement.

12. Procédé utile pour conférer des propriétés virucides à tout ou partie de la surface d'un support souple ou massique, ledit support étant choisi dans le groupe constitué des billets de banque, des cartes telles que des cartes à puce, des objets plastiques tels que les jouets, les claviers et souris d'ordinateurs, des écrans tactiles, des claviers, écrans et combinés de téléphones, des instruments de soins, médicaux ou de santé, des instruments de musique, des vêtements de travail, des outils et des tissus d'ameublement,
comprenant au moins l'étape consistant à appliquer une composition fluide apte à former un revêtement contenant de 0,1 % à 3 % en poids sec d'acide laurique par rapport au poids total de la composition,
ladite composition possédant une viscosité comprise entre 30 mPa.s et 40 Pa.s, à température et pression ambiante,
ladite composition comprenant en outre au moins un agent liant choisi parmi les résines, les cires et les gommes ;
et la génération de la monolaurine *in situ* en surface du support souple ou massique, lors de ladite application de ladite composition ;
la monolaurine étant synthétisée *in situ* par réaction d'acide laurique et de glycérol en présence d'un catalyseur, ledit catalyseur étant en particulier de type zéolite ou une lipase.

13. Procédé selon la revendication précédente, comprenant au moins les étapes consistant à:

    a) disposer d'un support souple ou massique dont la surface à traiter comporte au moins un catalyseur et/ou réactif apte à stimuler l'interaction entre l'acide laurique et le glycérol ; ledit catalyseur étant en particulier de type zéolite ou une lipase ;
    b) mettre en contact ladite surface avec une composition selon la revendication 12 contenant au moins de l'acide laurique et du glycérol ; et
    c) imposer à la surface traitée en étape b) un traitement thermique propice à la synthèse de la monolaurine ;

    lesdites étapes b) et c) pouvant être réalisées consécutivement ou simultanément.

14. Procédé selon la revendication 12 ou 13, dans lequel la composition comprend de 0,1 % à 2 % en poids sec, par exemple de 0,5 % à 1,5 % en poids sec, d'acide laurique par rapport à son poids total.

15. Procédé selon l'une quelconque des revendications 12 à 14, dans lequel la composition contient en outre un agent humectant, ledit agent humectant étant en particulier un polyol et plus particulièrement du glycérol.

16. Procédé selon la revendication précédente, dans lequel le rapport pondéral masse d'agent humectant sur masse d'acide laurique est au moins égal à 1.

17. Procédé selon l'une quelconque des revendications 12 à 16, dans lequel la composition est telle que défini selon l'une quelconque des revendications 6 à 10.

18. Procédé dans lequel une composition telle que définie en revendications 1 à 10 est déposée en surface d'un support par impression offset, par héliogravure, par flexographie, par surimpression flexographique, par gravure en taille-douce, par typographie ou par lithographie.

19. Objet **caractérisé en ce qu'**il est susceptible d'être obtenu par un procédé comprenant au moins une étape de revêtement en surface par une composition telle que définie en revendication 1 à 10.

**Patentansprüche**

1. Fluidzusammensetzung, geeignet zum Bilden einer Beschichtung, **gekennzeichnet dadurch, dass** sie 0,1% bis 3% im Trockengewicht in Bezug auf das Gesamtgewicht der Zusammensetzung von mindestens einem Viruzid natürlichen Ursprungs enthält, wobei das Viruzid natürlichen Ursprungs Monolaurin ist,
   wobei die Zusammensetzung eine Viskosität im Bereich zwischen 30 mPa·s und 40 Pa·s bei Umgebungstemperatur und - druck aufweist,
   wobei die Zusammensetzung unter anderem mindestens ein Bindemittel umfasst, das aus Harzen, Wachsen und Gummis ausgewählt ist.

2. Zusammensetzung nach Anspruch 1, **gekennzeichnet dadurch, dass** sie unter anderem mindestens ein bakteriostatisches und/oder bakterizides Biozid oder ein fungistatisches und/oder fungizides Biozid enthält.

3. Zusammensetzung nach einem der vorhergehenden Ansprüche, **gekennzeichnet dadurch, dass** sie 0,1% bis 2% im Trockengewicht, beispielsweise 0,5% bis 1,5% im Trockengewicht, des Viruzids bezüglich ihres Gesamtgewichts enthält.

4. Zusammensetzung nach einem der vorhergehenden Ansprüche, **gekennzeichnet dadurch, dass** sie unter anderem ein Feuchthaltemittel enthält, wobei das Feuchthaltemittel insbesondere ein Polyol und weiter insbesondere ein Glyzerol ist.

5. Zusammensetzung nach einem der vorhergehenden Ansprüche, **gekennzeichnet dadurch, dass** das Gewichtsverhältnis der Masse des Feuchthaltemittels zur Masse des Viruzids wenigstens gleich 1 ist.

6. Zusammensetzung nach einem der vorhergehenden Ansprüche, **gekennzeichnet dadurch, dass** sie vom Tinten- oder Lacktyp ist.

7. Zusammensetzung nach einem der vorhergehenden Ansprüche, **gekennzeichnet dadurch, dass** es sich um eine Tinte handelt, die mindestens ein Pigment und mindestens ein Bindemittel enthält, das aus einem Polyethylenwachs, einem Acrylharz und deren Mischungen ausgewählt ist.

8. Zusammensetzung nach einem der vorhergehenden Ansprüche, **gekennzeichnet dadurch, dass** sie eine Pigmentmenge enthält, die im Bereich von 0% und 60% im Gewicht, insbesondere zwischen 10% und 50% im Gewicht und darüber hinaus insbesondere zwischen 15% und 35% im Gewicht, bezüglich des Gesamtgewichts der Zusammensetzung liegt.

9. Zusammensetzung nach einem der Ansprüche 1 bis 6, **gekennzeichnet dadurch, dass** es sich um einen Lack handelt, der mindestens ein Bindemittel enthält, das insbesondere aus einem Polyethylenwachs, einem Acrylharz und deren Mischungen ausgewählt ist.

10. Fluidzusammensetzung nach einem der Ansprüche 1 bis 6, **gekennzeichnet dadurch, dass** es sich um einen Überdrucklack handelt, der mindestens ein Bindemittel enthält, das aus den zykloaliphatischen Epoxidharzen ausgewählt ist.

11. Verwendungsverfahren zum Aufbringen von viruziden Eigenschaften auf die gesamte Oberfläche eines flexiblen oder massigen Trägers oder einen Teil davon, umfassend mindestens den Schritt des Anwendens einer Zusammensetzung nach einem der Ansprüche 1 bis 10,
   wobei der Träger in der Gruppe ausgewählt wird, die gebildet wird durch Banknoten, Karten wie etwa Chipkarten, Plastikobjekte wie etwa Spielzeug, Tastaturen und Mäuse von Computern, berührungsempfindliche Bildschirme, Tastaturen, Bildschirme und Hörer von Telefonen, Instrumente für Pflege, Medizin oder Gesundheit, Musikinstrumente, Arbeitskleidung, Werkzeuge und Möbelstoffe.

12. Verwendungsverfahren zum Aufbringen von viruziden Eigenschaften auf die gesamte Oberfläche eines flexiblen oder massigen Trägers oder einen Teil davon,
   wobei der Träger in der Gruppe ausgewählt wird, die gebildet wird durch Banknoten, Karten wie etwa Chipkarten, Plastikobjekte wie etwa Spielzeug, Tastaturen und Mäuse von Computern, berührungsempfindliche Bildschirme, Tastaturen, Bildschirme und Hörer von Telefonen, Instrumente für Pflege, Medizin oder Gesundheit, Musikinstrumente, Arbeitskleidung, Werkzeuge und Möbelstoffe,

umfassend mindestens den Schritt des Anwendens einer Fluidzusammensetzung, die zur Bildung einer Beschichtung geeignet ist und 0,1% bis 3% im Trockengewicht von Laurinsäure bezüglich des Gesamtgewichts der Zusammensetzung enthält,

wobei die Zusammensetzung eine Viskosität im Bereich von 30 mPa·s und 40 Pa·s bei Umgebungstemperatur und -druck aufweist,

wobei die Zusammensetzung unter anderem mindestens ein Bindemittel umfasst, das aus den Harzen, den Wachsen und den Gummis ausgewählt ist;

und die Erzeugung des Monolaurins *in situ* auf der Oberfläche des flexiblen oder massigen Trägers nach der Anwendung der Zusammensetzung;

wobei das Monolaurin *in situ* durch Reaktion der Laurinsäure und von Glyzerol bei Vorhandensein eines Katalysators synthetisiert wird, wobei der Katalysator insbesondere vom Zeolithtyp oder eine Lipase ist.

13. Verfahren nach dem vorhergehenden Anspruch, umfassend mindestens die folgenden Schritte:

a) Bereitstellen eines flexiblen oder massigen Trägers, dessen zu behandelnde Oberfläche mindestens einen Katalysator und/oder Reaktionsmittel aufweist, der bzw. das zum Stimulieren der Interaktion der Laurinsäure und des Glyzerols geeignet ist, wobei der Katalysator insbesondere vom Zeolithtyp oder eine Lipase ist;

b) In-Kontakt-Bringen der Oberfläche mit einer Zusammensetzung nach Anspruch 12, die mindestens die Laurinsäure und Glyzerol enthält, und

c) Unterziehen der in Schritt b) behandelten Oberfläche einer Wärmebehandlung, die für die Synthese des Monolaurins günstig ist;

wobei die Schritte b) und c) aufeinanderfolgend oder gleichzeitig ausgeführt werden können.

14. Verfahren nach Anspruch 12 oder 13, bei dem die Zusammensetzung 0,1% bis 2% im Trockengewicht, beispielsweise 0,5% bis 1,5% im Trockengewicht, Laurinsäure bezüglich ihres Gesamtgewichts enthält.

15. Verfahren nach einem der Ansprüche 12 bis 14, bei dem die Zusammensetzung unter anderem ein Feuchthaltemittel enthält, wobei das Feuchthaltemittel insbesondere ein Polyol und darüber hinaus insbesondere aus Glyzerol ist.

16. Verfahren nach dem vorhergehenden Anspruch, bei dem das Gewichtsverhältnis der Masse des Feuchthaltemittels zur Masse der Laurinsäure wenigstens gleich 1 ist.

17. Verfahren nach einem der Ansprüche 12 bis 16, bei dem die Zusammensetzung so wie in einem der Ansprüche 6 bis 10 definiert beschaffen ist.

18. Verfahren, bei dem eine Zusammensetzung nach einem der Ansprüche 1 bis 10 auf einem Träger durch Offsetdruck, durch Lichtdruck, durch Flexographie, durch flexographischen Aufdruck, durch Stichdruck, durch Typographie oder durch Lithographie aufgebracht wird.

19. Gegenstand, **gekennzeichnet dadurch, dass** er durch ein Verfahren erzeugt werden kann, das mindestens einen Schritt der Oberflächenbeschichtung mit einer Zusammensetzung nach einem der Ansprüche 1 bis 10 umfasst.

**Claims**

1. A fluid composition capable of forming a coating, **characterized in that** it contains from 0.1% to 3% by dry weight, relative to the total weight of the composition, of at least one virucide of natural origin, said virucide of natural origin being monolaurin, said composition having a viscosity between 30 mPa.s and 40 Pa.s, at room temperature and ambient pressure,

said composition comprising, in addition, at least one binder chosen from resins, waxes and gums.

2. The composition as claimed in claim 1, **characterized in that** it also contains at least one bacteriostatic and/or bactericidal biocide or a fungistatic and/or fungicidal biocide.

3. The composition as claimed in any one of the preceding claims, **characterized in that** it contains from 0.1% to 2% by dry weight, for example from 0.5% to 1.5% by dry weight, of said virucide relative to its total weight.

4. The composition as claimed in any one of the preceding claims, **characterized in that** it also contains a humectant, said humectant being in particular a polyol and more particularly glycerol.

5. The composition as claimed in any one of the preceding claims, **characterized in that** the weight ratio of the mass of humectant to the mass of virucide is at least equal to 1.

6. The composition as claimed in any one of the preceding claims, **characterized in that** it is of ink or varnish type.

7. The composition as claimed in any one of the preceding claims, **characterized in that** it is an ink containing at least one pigment and at least one binder in particular chosen from a polyethylene wax, an acrylic resin and mixtures thereof.

8. The composition as claimed in any one of the preceding claims, **characterized in that** it contains an amount of pigments between 0% and 60% by weight, especially between 10% and 50% by weight, and in particular between 15% and 35% by weight, relative to the total weight of said composition.

9. The composition as claimed in any one of claims 1 to 6, **characterized in that** it is a varnish containing at least one binder in particular chosen from a polyethylene wax, an acrylic resin and mixtures thereof.

10. The fluid composition as claimed in any one of claims 1 to 6, **characterized in that** it is an overprint varnish containing at least one binder in particular chosen from cycloaliphatic epoxy resins.

11. A process useful for imparting virucidal properties to all or part of the surface of a flexible or solid support comprising at least the step that consists in applying a composition as defined in claims 1 to 10,
said support being selected in the group consisting in banknotes, cards such as chip cards, plastic articles such as toys, computer keyboards and mice, touch screens, telephone keypads, screens and handsets, care, medical or health instruments, musical instruments, uniforms, tools and upholstery fabrics.

12. A process useful for imparting virucidal properties to all or part of the surface of a flexible or solid support,
said support being selected in the group consisting in banknotes, cards such as chip cards, plastic articles such as toys, computer keyboards and mice, touch screens, telephone keypads, screens and handsets, care, medical or health instruments, musical instruments, uniforms, tools and upholstery fabrics,
comprising at least the application of a fluid composition capable of forming a coating, containing from 0.1% to 3% by dry weight, of lauric acid, relative to the total weight of the composition,
said composition having a viscosity between 30 mPa.s and 40 Pa.s, at room temperature and ambient pressure,
said composition comprising, in addition, at least one binder chosen from resins, waxes and gums,
and the generation of monolaurin *in situ* on the surface of the flexible or solid support, during said application of said composition,
the monolaurin being synthesized *in situ* by reaction of lauric acid and glycerol in the presence of a catalyst, said catalyst being in particular of zeolite-type or a lipase.

13. The process as claimed in the preceding claim, comprising at least the steps that consist in:

    a) using a flexible or solid support having a surface to be treated that comprises at least one catalyst and/or reactant capable of stimulating the interaction between lauric acid and glycerol; said catalyst being in particular of zeolite-type or a lipase;
    b) bringing said surface into contact with a composition as claimed in claim 1 containing at least lauric acid and glycerol; and
    c) subjecting the surface treated in step b) to a heat treatment conducive to the synthesis of monolaurin;

    it being possible for said steps b) and c) to be carried out consecutively or simultaneously.

14. The process as claimed in claim 12 or 13, wherein the composition comprises from 0.1% to 2% by dry weight, for example from 0.5% to 1.5% by dry weight, of lauric acid relative to its total weight.

15. The process as claimed in any one of claims 12 to 14, wherein the composition also contains a humectant, said humectant being in particular a polyol and more particularly glycerol.

**16.** The process as claimed in the preceding claim, wherein the weight ratio of the mass of humectant to the mass of virucide is at least equal to 1.

**17.** The process as claimed in any one of claims 12 to 16, wherein the composition is as defined in any one of claims 6 to 10.

**18.** A process wherein a composition as defined in claims 1 to 10 is deposited on the surface of a support by offset printing, gravure printing, by flexography, by flexographic overprinting, by intaglio printing, by typography or by lithography.

**19.** Articles **characterized in that** they may be obtained by a process comprising at least one step of coating the surface with a composition as defined in claims 1 to 10.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5968538 A

- US 4662403 A

**Littérature non-brevet citée dans la description**

- **CLARKE et al.** *Journal of Medical Microbiology,* vol. 49, 719-723
- **THORMAR et al.** *Antimicrobial Agents and Chemotherapy,* 01 Janvier 1987, 27-31

- **C.C.B., DA SILVA M.A.P. ; LANGONE M.A.P.** Enzymatic synthesis of monolaurine. *Applied biochemistry and Biotechnology,* 2004, vol. 113 (116), 433-445